# EUROPEAN PATENT APPLICATION

(11) **EP 4 261 541 A1**
(43) Date of publication of application: **18.10.2023**
(21) Application number: 21903370.1
(22) Date of filing: 06.12.2021
(51) Int. Cl.: G01N 33/543, G01N 33/569

(54) **SENSITIZER FOR IMMUNOCHROMATOGRAPHIC ASSAYS, AND ASSAY**

(30) Priority: 11.12.2020 JP 2020206166; 19.01.2021 JP 2021006659
(71) Applicant: NOF Corporation, Shibuya-ku Tokyo 150-6019 (JP)
(72) Inventor: KOBAYASHI, Koh, Kawasaki-shi, Kanagawa 210-0865 (JP); MATSUDA, Masaru, Kawasaki-shi, Kanagawa 210-0865 (JP)
(74) Representative: J A Kemp LLP
(86) International application number: PCT/JP2021/044765
(87) International publication number: WO 2022/124270

(57) **Abstract**

The present invention provides a sensitizer for immunochromatographic assay capable of detecting novel coronavirus (SARS-CoV-2) IgM antibodies and/or IgG antibodies with high sensitivity, and an assay using the sensitizer. Specifically, the present invention provides a sensitizer for immunochromatographic assay for novel coronavirus (SARS-CoV-2) IgM antibodies and/or IgG antibodies as assay target substances, that contains a polymer represented by the following formula [4] : 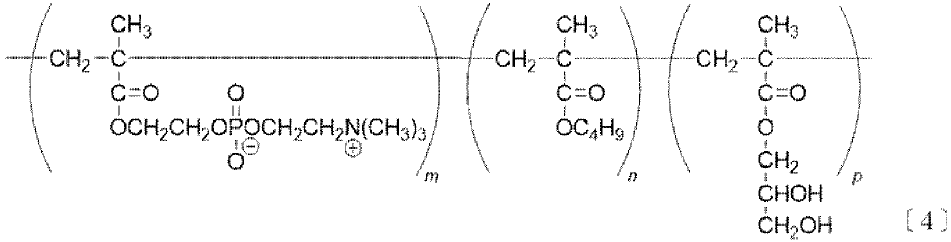 wherein m, n, and p are each a constitutional unit number, and m:n:p is 100 to 30:0 to 70:0 to 50.

## Description

### [Technical Field]

The present invention relates to a sensitizer for immunochromatographic assay for novel coronavirus (SARS-CoV-2) IgM antibodies and/or IgG antibodies as assay target substances, and an immunochromatographic assay in the co-presence of the sensitizer for novel coronavirus (SARS-CoV-2) IgM antibodies and/or IgG antibodies as assay target substances.

### [Background Art]

Immunochromatography is a method that combines a chromatographic method using membrane capillarity and an immunological method using antigen-antibody reaction. Immunochromatography is widely used as a clinical diagnosis method because it has high specificity due to the antigen-antibody reaction, is easy to operate, can be tested on the spot without requiring a place or special facility, and permits visual observation of the results (Patent Literature 1).

Immunochromatography is advantageous in that the assay is easy, but the accuracy of detection sensitivity is low, and positive samples are often judged as false negatives. Thus, an immunochromatographic assay with higher sensitivity is desired.

To date, various methods have been studied for the purpose of establishing a highly sensitive immunochromatographic assay. For example, Patent Literature 2 discloses a method of increasing sensitivity by changing the addition position of immunochromatographic reagents. Also, Patent Literature 3 discloses a method of increasing sensitivity by improving the materials of the membrane.

Furthermore, a method of increasing sensitivity by adding an additive to sample diluents used in conventional immunochromatography is known. For example, Patent Literatures 4, 5, and 6 disclose adding bovine serum albumin (BSA), a polymer having a phosphoryl choline group, and hyaluronic acid to sample diluents. These disclosures are advantageous in that the addition position of reagents does not need to be changed and that conventional membranes for immunochromatography can be used.

However, the above-mentioned Patent Literatures do not disclose or suggest sensitizers used in methods for highly sensitive assay of novel coronavirus (SARS-CoV-2) IgM antibodies and IgG antibodies.

In particular, the novel coronavirus (SARS-CoV-2) IgM antibody is an antibody produced in the early stages of infection with the novel coronavirus (SARS-CoV-2). If the IgM antibody can be detected with high sensitivity, the spread of infection can be suppressed because the infected person can be isolated at the early stages of infection. In addition, since the treatment can be started early, the effect of the treatment can be enhanced. Thus, a sensitizer that enables highly sensitive detection of IgM antibody is considered to be highly useful.

Furthermore, the novel coronavirus (SARS-CoV-2) IgG antibody is an antibody that is produced over a long time in the body. If the IgG antibody can be detected with high sensitivity, infected people in the late stage of infection can be identified. A sensitizer that enables highly sensitive detection of IgG antibodies produced over a long period of time is considered to be highly useful in identifying people infected with novel coronavirus, many of whom are unaware of their infection.

### [Citation List]

### [Patent Literature]

[PTL 1]
   JP-A-H01-063865
[PTL 2]
   JP-A-2014-66674
[PTL 3]
   JP-A-2014-62820
[PTL 4]
   JP-A-2008-292326
[PTL 5]
   JP-A-2008-058334
[PTL 6]
   JP-A-2003-344413

### [Summary of Invention]

### [Technical Problem]

The present invention aims to provide a sensitizer for immunochromatographic assay that can detect novel coronavirus (SARS-CoV-2) IgM antibodies and/or IgG antibodies with high sensitivity, and an assay using the sensitizer.

### [Solution to Problem]

The present inventors have conducted intensive studies in an attempt to find a compound capable of detecting novel coronavirus (SARS-CoV-2) IgM antibodies and/or IgG antibodies with high sensitivity and found that a polymer or a copolymer represented by the following formula [1], [3], or [4] and having a group having a phosphorylcholine analogous group as a side chain has the desired properties, which resulted in the completion of the present invention.

That is, the present invention includes the following.
[1] A sensitizer for immunochromatographically assaying novel coronavirus (SARS-CoV-2) IgM antibodies and/or IgG antibodies as assay target substances, comprising a polymer represented by the following formula [4]: 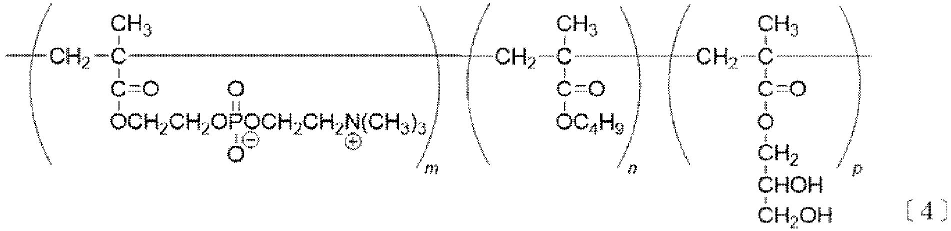 wherein m, n, and p are each a constitutional unit number, and m:n:p is 100 to 30:0 to 70:0 to 50.
[2] A sensitizer for immunochromatographically assaying novel coronavirus (SARS-CoV-2) IgM antibodies and/or IgG antibodies as assay target substances, comprising a polymer represented by the following formula [3]: wherein m and n are each a constitutional unit number, and m:n is 100 to 30:0 to 70.
[3] A sample diluent for immunochromatographically assaying novel coronavirus (SARS-CoV-2) IgM antibodies and/or IgG antibodies as assay target substances, comprising the sensitizer for immunochromatographic assay of [1].
[4] A method for immunochromatographically assaying novel coronavirus (SARS-CoV-2) IgM antibodies and/or IgG antibodies as assay target substances, comprising conducting an antigen-antibody reaction in the presence of the sensitizer for immunochromatographic assay of [1].
[5] An instrument for immunochromatographically assaying novel coronavirus (SARS-CoV-2) IgM antibodies and/or IgG antibodies as assay target substances, comprising the sensitizer for immunochromatographic assay of [1].
[6] An instrument for immunochromatographically assaying novel coronavirus (SARS-CoV-2) IgM antibodies and/or IgG antibodies as assay target substances, comprising the sensitizer for immunochromatographic assay of [1], wherein the sensitizer is carried on a development membrane, a sample pad, or a conjugate pad.
[7] A test kit for novel coronavirus infection (COVID-19), comprising the sensitizer for immunochromatographic assay of [1] and a substance that specifically binds to a novel coronavirus (SARS-CoV-2) IgM antibody and/or IgG antibody.
[8] A method for testing a test subject for the possibility of being infected with a novel coronavirus (COVID-19), comprising measuring novel coronavirus (SARS-CoV-2) IgM antibodies and/or IgG antibodies in a biological sample derived from the test subject by conducting an antigen-antibody reaction in the presence of the sensitizer for immunochromatographic assay of [1] .
[9] A method for treating a novel coronavirus infection (COVID-19), comprising the following steps (1) and (2):
   (1) a step of measuring a novel coronavirus (SARS-CoV-2) by conducting an antigen-antibody reaction in the presence of a sensitizer for immunochromatographic assay comprising a polymer represented by the following formula [4], and
   (2) a step of treating a patient with a novel coronavirus infection (COVID-19) based on the assay results of the above-mentioned (1):

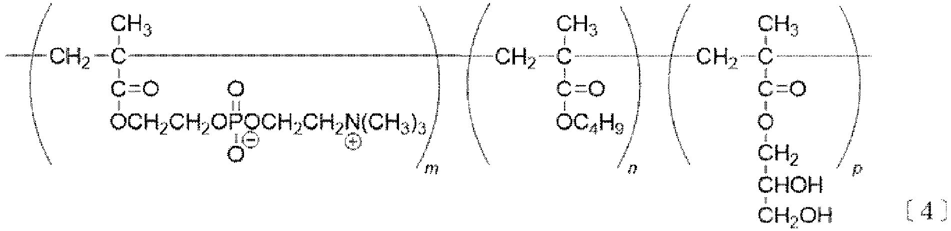

      wherein m, n, and p are each a constitutional unit number, and m:n:p is 100 to 30:0 to 70:0 to 50.
[10] A kit for the treatment of a novel coronavirus infection (COVID-19), comprising a sensitizer for immunochromatographic assay comprising a polymer represented by the following formula [4] and a therapeutic agent for the novel coronavirus infection (COVID-19): 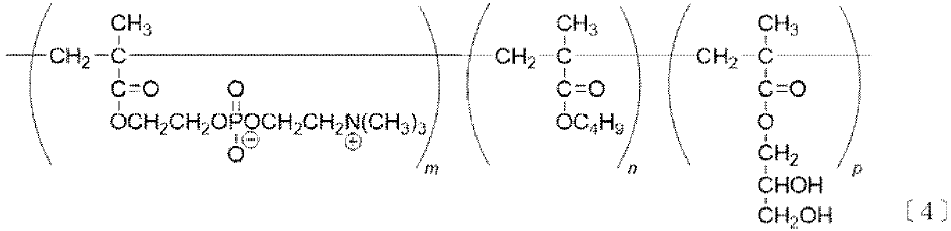 wherein m, n, and p are each a constitutional unit number, and m:n:p is 100 to 30:0 to 70:0 to 50.
[11] A sensitizer for immunochromatographically assaying novel coronavirus (SARS-CoV-2) IgM antibodies and/or IgG antibodies as assay target substances, comprising a polymer represented by the following formula [4] and a copolymer represented by the following formula [5]: 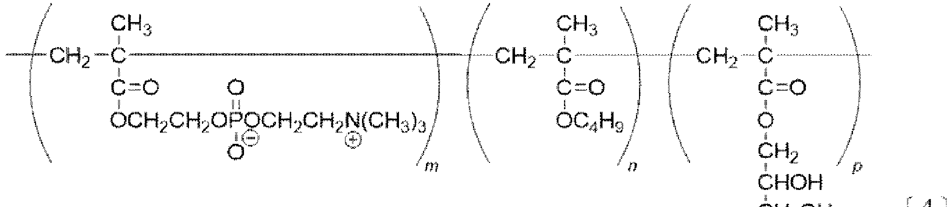 wherein m, n, and p are each a constitutional unit number, and m:n:p is 100 to 30:0 to 70:0 to 50,
   wherein R in the number of q are each independently a hydrogen atom or a cation, m and q are each a constitutional unit number, and m:q is 99 to 20:1 to 80.

### [Advantageous Effects of Invention]

A method of the present invention using the sensitizer for immunochromatographically assaying novel coronavirus (SARS-CoV-2) IgM antibodies and/or IgG antibodies as assay target substances can detect novel coronavirus (SARS-CoV-2) IgM antibodies and/or IgG antibodies with high sensitivity, as compared with conventional immunochromatographic assays for detecting novel coronavirus (SARS-CoV-2) IgM antibodies and/or IgG antibodies.

### [Description of Embodiments]

A sensitizer for immunochromatographically assaying novel coronavirus (SARS-CoV-2) IgM antibodies and/or IgG antibodies as assay target substances, a sample diluent containing the sensitizer which is for immunochromatographic assay of the above-mentioned antibodies as assay target substances, a method for immunochromatographically assaying the above-mentioned antibodies as assay target substances, including conducting an antigen-antibody reaction in the presence of the sensitizer, an instrument containing the sensitizer which is for immunochromatographic assay of the above-mentioned antibodies as assay target substances, and a method for treating a novel coronavirus infection (COVID-19), including a step of conducting an antigen-antibody reaction in the presence of the sensitizer, of the present invention are described below.

In a method using the sensitizer for immunochromatographic assay of the present invention, the assay target substance is a novel coronavirus (SARS-CoV-2) IgM antibody and/or IgG antibody, but other assay target substances can also be detected. The sensitizer for immunochromatographic assay of the present invention is characterized in that it can detect the above-mentioned antibodies with high sensitivity.

In the present specification, the novel coronavirus refers to the severe acute respiratory syndrome coronavirus 2 (SARS-CoV-2); a coronavirus strain that causes novel coronavirus infection (COVID-19) (also referred to as coronavirus disease 2019), which is the respiratory disease that caused the COVID-19 pandemic. Each SARS-CoV-2 virion is 50-200 nanometers in diameter, and SARS-CoV-2, like other coronaviruses, has four structural proteins known as S (spike), E (envelope), M (membrane), and N (nucleocapsid) proteins. The N protein holds the RNA genome, and forms a viral envelope together with the S, E, and M proteins. Spike proteins are proteins that enable viruses to attach to and fuse with host cell membranes. SARS-CoV-2 is transmitted from human to human, and transmission was initially assumed to occur primarily via respiratory droplets from coughs and sneezes within a certain area. It has also been suggested that the virus may also be airborne, since aerosols can cause viral infection.

The polymer to be used as the sensitizer for immunochromatographically assaying novel coronavirus (SARS-CoV-2) IgM antibodies and/or IgG antibodies as assay target substances of the present invention is represented by the following formula [3]:

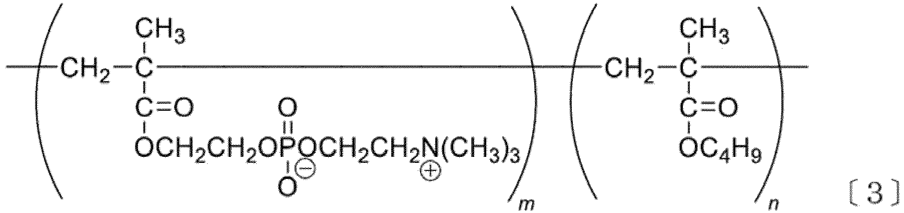

wherein m and n are each a constitutional unit number, and m:n is 100 to 30:0 to 70.

In one embodiment, the polymer represented by the above-mentioned formula [3] is a polymer having only a constitutional unit based on a monomer represented by the following formula [2] {MPC: 2-(methacryloyloxy)ethyl 2'-(trimethylammonio)ethyl phosphate}:

That is, the polymer is represented by the following formula [1]: 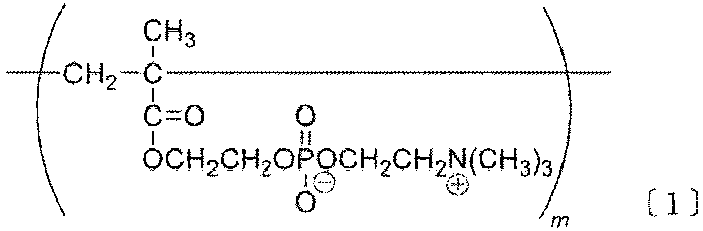 wherein m is a constitutional unit number.

The polymer represented by the above-mentioned formula [1] is preferably a water-soluble polymer having a weight average molecular weight of 100,000 to 2,000,000, more preferably 500,000 to 1,500,000.

In another embodiment, the polymer represented by the above-mentioned formula [3] is a copolymer having a constitutional unit based on 2-(methacryloyloxy)ethyl 2'-(trimethylammonio)ethyl phosphate (MPC) represented by the above-mentioned formula [2], and a constitutional unit based on butyl methacrylate.

The kind of the copolymer is not particularly limited, and may be a random copolymer or a block copolymer, with preference given to a random copolymer.

In the copolymer, the ratio of the constitutional unit number based on 2-(methacryloyloxy)ethyl 2'-(trimethylammonio)ethyl phosphate (MPC) and the constitutional unit number based on butyl methacrylate (m:n) is preferably 90 to 30:10 to 70.

The copolymer is preferably a water-soluble copolymer having a weight average molecular weight of 10,000 to 2,000,000, more preferably 500,000 to 1,500,000.

The polymer represented by the above-mentioned formula [3] may further contain other constitutional units as long as the effect of the present Invention is not impaired. Examples of other constitutional unit include constitutional units based on monomers such as alkyl(meth)acrylates, cyclic alkyl(meth)acrylates, aromatic group-containing (meth)acrylates, hydroxy group-containing (meth)acrylates, and the like. Among these monomers, a hydroxy group-containing (meth)acrylate is preferred. As the hydroxy group-containing (meth)acrylate, 2-hydroxy(meth)acrylate or glycerol (meth)acrylate is preferred, and glycerol methacrylate is particularly preferred.

The polymer represented by the above-mentioned formula [3] optionally further containing a constitutional unit based on glycerol methacrylate is represented by the following formula [4]: 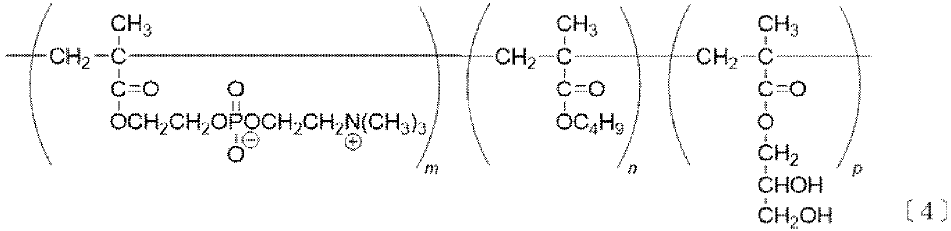 wherein m, n, and p are each a constitutional unit number, and m:n:p is 100 to 30:0 to 70:0 to 50.

In the polymer represented by the above-mentioned formula [4], the ratio of the constitutional unit number based on 2-(methacryloyloxy)ethyl 2'-(trimethylammonio)ethyl phosphate (MPC) represented by the above-mentioned formula [2], the constitutional unit number based on butyl methacrylate, and the constitutional unit number based on glycerol methacrylate (m:n:p) is preferably 30 to 50:20 to 50:20 to 50.

The polymer represented by the above-mentioned formula [4] is preferably a water-soluble copolymer having a weight average molecular weight of 10,000 to 100,000, more preferably 20,000 to 50,000.

When the polymer represented by the above-mentioned formula [4] is a copolymer, the kind of the copolymer is not particularly limited and may be a random copolymer or a block copolymer, and a random copolymer is preferred.

The polymer represented by the above-mentioned formula [3] or [4] which is used as the sensitizer for immunochromatographc assay is preferably exemplified by, but is not particularly limited to, those described in the following examples.

The sensitizer of the present invention for immunochromatographic assay may further contain a copolymer represented by the following formula [5], in addition to the polymer represented by the above-mentioned formula [3] or [4]: wherein R in the number of q are each independently a hydrogen atom or a cation, m and q are each a constitutional unit number, and m:q is 99 to 20:1 to 80.

In the above-mentioned formula, R in the number of q are each independently a hydrogen atom or a cation. Here, cation is exemplified by sodium ion, potassium ion, ammonium ion, and the like, and the R in the number of q may be the same or different. R is preferably a hydrogen atom.

In the above-mentioned formula, m and q are each a constitutional unit number, and m:q is 99 to 20:1 to 80, preferably 90 to 30:10 to 70.

The copolymer represented by the above-mentioned formula [5] is preferably a water-soluble copolymer having a weight average molecular weight of 10,000 to 5,000,000, preferably 50,000 to 2,000,000.

The copolymer represented by the above-mentioned formula [5] is a copolymer having a constitutional unit based on a monomer represented by the above-mentioned formula [2] {MPC: 2-(methacryloyloxy)ethyl 2'-(trimethylammonio)ethyl phosphate}, and a constitutional unit based on methacrylic acid or a salt thereof.

The kind of the copolymer is not particularly limited, and may be a random copolymer or a block copolymer, with preference given to a random copolymer.

Examples of the methacrylic acid or a salt thereof include methacrylic acid, sodium methacrylate, potassium methacrylate, and ammonium methacrylate. Among these, methacrylic acid is preferred.

The copolymer represented by the above-mentioned formula [5] is preferably exemplified by those described in the following examples, but is not particularly limited.

The content of the copolymer represented by the above-mentioned formula [5] in the sensitizer for immunochromatographic assay is not particularly limited, and is 0.1 to 5.0 w/v%, preferably 0.5 to 1.0 w/v%.

The polymer or copolymer to be used as the sensitizer for immunochromatographic assay of the present invention may be used by dissolving in various reagents or samples used in immunochromatographic assay, or may be carried in an immunochromatographic assay kit. Preferably, it is used as a sample diluent.

In addition, the immunochromatographic assay of the present invention is generally composed of a sample diluent and a test strip as an immunochromatographic assay kit to be used.

The sample diluent is a diluent suitable for performing assay by immunochromatography, and may contain buffer components such as Tris buffering agent, phosphate buffering agent, Veronal buffering agent, borate buffering agent, and Good's buffering agent, stabilizing components such as albumin, globulin, casein, serum, water-soluble gelatin, surfactant, saccharides, and chelating agent, and preservative components such as salicylic acid, benzoic acid, and sodium azide.

When the sensitizer of the present invention is used by dissolving in a sample diluent, the concentration thereof during an antigen-antibody reaction is not particularly limited, and is generally 0.1 to 5.0 w/v%, preferably 0.5 to 1.0 w/v%. In one embodiment, the concentration of the sensitizer of the present invention in a sample diluent is generally 0.1 to 5.0 wt%, preferably 0.5 to 1.0 wt%.

A test strip generally contains at least a first substance capable of antigen-antibody reaction with a first antigenic determinant of a sample, a labeled second substance capable of antigen-antibody reaction with the second antigenic determinant of the aforementioned sample, and a membrane carrier, and is configured such that the aforementioned first substance is immobilized in advance at a predetermined position on the aforementioned membrane carrier to form a trapping site, and the aforementioned second substance is disposed so as to be chromatographically developable on the aforementioned carrier at a position isolated from the aforementioned trapping site. In one embodiment, when the sample is an antibody (including IgM antibodies and/or IgG antibodies), the above-mentioned first substance and/or the second substance can be an antigen for the antibody.

A typical configuration of a test strip for an immunochromatography is, for example, a configuration in which a sample supply part for supplying a sample (hereinafter sometimes to be referred to as a sample pad), a conjugate pad for placing a conjugate, a development membrane that has a detection part in which a trapping reagent such as an antibody is immobilized in a line and that traps the above-mentioned immunocomplex by developing same by mobile phase, and an absorption pad for absorbing the sample that has been developed on the development membrane downstream of the detection part are disposed.

Specific examples of the material suitable for the sample pad include, but are not limited to, glass fiber, acrylic fiber, hydrophilic polyethylene material, polyester, dry paper, paper pulp, cellulose fiber, and fabric. The sample pad may contain, as long as the purpose of the present invention is not deviated and the reaction system is not affected, blocking reagents and buffer components generally used as necessary and, when the sample is blood, blood coagulants, and the like. In that case, they only need to be contained in at least a part of the sample pad, and may also be contained in the entire sample pad.

In one embodiment, the conjugate pad is made of a pad-shaped porous material in which a sample that has passed through the sample pad can be developed and which can retain the conjugate. The conjugate is retained in a part or the whole of the conjugate pad. Examples of the porous material constituting the conjugate pad include nonwoven fibers such as paper, cellulose mixture, cellulose fiber, nitrocellulose, polyester, acrylonitrile copolymer, glass (glass fiber), and rayon.

As the test strip for immunochromatographic assay, a commercially available product may be used or a test strip produced by a known method may also be used. When it is produced by a known method, the base material used in the above-mentioned development membrane may be those generally used in this field, and preferred examples thereof include cellulose, nitrocellulose, and nylon.

Examples of the instrument for immunochromatographically assaying novel coronavirus (SARS-CoV-2) IgM antibodies and/or IgG antibodies as assay target substances and containing the sensitizer of the present invention include, but are not particularly limited to, (1) one composed of a development membrane, (2) one composed of a sample-labeling part and a development membrane, and formed such that antibodies can move between the sample-labeling part and the development membrane are movable by capillarity, (3) one composed of a sample-labeling part, a development membrane, and a liquid-absorbing part, and formed such that antibodies can move along the sample-labeling part, the development membrane, and the liquid-absorbing part in this order by capillarity, (4) one composed of a sample-dropping part, a sample-labeling part, a development membrane, and a liquid-absorbing part, and formed such that antibodies can move along the sample-dropping part, the sample-labeling part, the development membrane, and the liquid-absorbing part in this order by capillarity.

When the sensitizer of the present invention is used by being carried by the aforementioned instrument for immunochromatographic assay, the amount thereof varies depending on the place where it is carried, the kind of the sensitizer to be used, the kind of the substance to be used for the assay, the labeling substance to be used, and the like. For example, it can be carried on a development membrane, a sample-labeling part, a sample-dropping part such as a sample pad or a conjugate pad, and the like, of an instrument for immunochromatographic assay. When the sensitizer of the present invention is carried by the development membrane and the sample-labeling part of the instrument for immunochromatographic assay, the amount of the sensitizer contained per unit area (cm²) is generally 0.01 µg to 10 mg, preferably 0.1 µg to 4 mg, more preferably 1 to 800 µg. When the sensitizer of the present invention is carried by the sample-dropping part and the sample-dropping part is a sample pad, the sensitizer of the present invention to be carried is generally 0.01 µg to 50 mg, preferably 100 µg to 20 mg, more preferably 1 to 10 mg. When the sample-dropping part is a conjugate pad, the sensitizer of the present invention to be carried is generally 0.01 µg to 10 mg, preferably 0.1 µg to 4 mg, more preferably 1 to 800 µg. When two or more polymers or copolymers having different weight average molecular weights and ratios of the number of structural units are carried, the above-mentioned amount of the sensitizer is the amount for each. In addition, the area to be carried varies depending on the kind and size of the instrument for immunochromatographic assay to be used and the amount of the assay sample. In the case of the development membrane of the instrument for immunochromatographic assay, it is generally 5 to 600, preferably 5 to 150, of the total area and in the case of the sample-dropping part, it is generally 5 to 400, preferably 10 to 200, of the total area.

In addition, the sensitizer of the present invention may be used by being carried on a development membrane, a sample-labeling part, a sample-dropping part such as sample pad, conjugate pad, or the like, and the like of an instrument for immunochromatographic assay, which is a constituent element of a commercially available kit. Examples of such instrument for immunochromatographic assay, which is a constituent element of a commercially available kit, include instruments for immunochromatographic assays that are constituent elements of Novel Coronavirus (SARS-CoV-2) IgM Test Kit (Colloidal Gold) (manufactured by Ray Biotec.), Novel Coronavirus (SARS-CoV-2) IgG Test Kit (Colloidal Gold) (manufactured by Ray Biotec.), novel coronavirus IgM/IgG antibody test kit COVID-19 IgM/IgG Plus (manufactured by Malcom Co., Ltd.), novel coronavirus (SARS-CoV-2)antibody test kit (IgM)RF-NC001 (manufactured by Kurabo Industries Ltd.), novel coronavirus (SARS-CoV-2)antibody test kit (IgG)RF-NC002 (manufactured by Kurabo Industries Ltd.), SARS-CoV-2 Antibody Test (colloidal gold immunochromatography) (manufactured by Lepu Medical Technology), and the like.

The present invention provides a test kit for novel coronavirus infection (COVID-19), containing the sensitizer for immunochromatographic assay of the present invention and a substance that specifically binds to a novel coronavirus (SARS-CoV-2) IgM antibody and/or IgG antibody.

Being "specific" means that the affinity for novel coronavirus (SARS-CoV-2) IgM antibodies and/or IgG antibodies is higher than the affinity for other substances (in one embodiment, IgM antibodies and/or IgG antibodies against antigens other than proteins of the novel coronavirus (SARS-CoV-2) are not included in of the "other substance"). The specifically binding substance binds to a novel coronavirus (SARS-CoV-2) IgM antibody and/or IgG antibodt with a KD of, for example, about 10⁻⁶, 10⁻⁷, 10⁻⁸, 10⁻⁹, 10⁻¹⁰ or less. In addition, the specifically binding substance binds to a novel coronavirus (SARS-CoV-2) IgM antibody and/or IgG antibody with a KD of 10⁻¹⁴, 10⁻¹³, 10⁻¹² or above.

The aforementioned substance preferably isolated or purified. The "isolated or purified" means that an operation has been performed to remove components other than the desired component from the natural state. The purity of the aforementioned isolated or purified substance (ratio of the weight of the aforementioned substance to the total protein weight) is generally 500 or more, preferably 70% or more, more preferably 90% or more, most preferably 950 or more (for example, substantially 1000).

The aforementioned substance may be directly or indirectly labeled with a labeling substance. Examples of the labeling substance include fluorescent substances (e.g., FITC, rhodamine), radioactive substances (e.g., ¹⁴C, ³H, ¹²⁵I), enzymes (e.g., alkali phosphatase, peroxidase), colored particles (e.g., metal colloid particles, colored latex), biotin, and the like.

In the present invention, examples of the "substance that specifically binds to a novel coronavirus (SARS-CoV-2) IgM antibody and/or IgG antibody" include antigens for the novel coronavirus (SARS-CoV-2) IgM antibody and/or IgG antibody, and antibodies, nucleic acids (e.g., aptamer), and the like that specifically bind to the novel coronavirus (SARS-CoV-2) IgM antibody and/or IgG antibody, and antigens for the novel coronavirus (SARS-CoV-2) IgM antibody and/or IgG antibody, and anti-novel coronavirus (SARS-CoV-2) IgM antibodies and/or IgG antibodies are preferred.

The aforementioned antigen is not limited as long as it is an antigen for the novel coronavirus (SARS-CoV-2) IgM antibodies and/or IgG antibodies that are the assay target in the present invention. Examples thereof include novel coronavirus S (spike) protein, E (envelope) protein, M (membrane) protein, and N (nucleocapsid) protein.

When a recombinant novel coronavirus (SARS-CoV-2) protein is used as the antigen, the recombinant novel coronavirus (SARS-CoV-2) protein can be produced, for example, by the following method. A polynucleotide encoding the amino acid sequence of the novel coronavirus (SARS-CoV-2) protein is incorporated into an appropriate expression vector, the vector is inserted into an appropriate host for transformation, and the desired recombinant novel coronavirus (SARS-CoV-2) protein can be obtained from a homogenate of the transformed cells. The above-mentioned host cell is not particularly limited, and various host cells conventionally used in genetic engineering methods, such as Escherichia coli, Bacillus subtilis, yeast, plant or animal cell, and the like, can be used.

The novel coronavirus (SARS-CoV-2) protein is a recombinant protein produced from the above-mentioned transformant, or may be isolated or purified from a natural novel coronavirus (SARS-CoV-2) that produces the protein by a protein separation and purification technique known per se. It may also be a protein chemically synthesized or biochemically synthesized in a cell-free translation system.

In one embodiment, the above-mentioned "anti-novel coronavirus (SARS-CoV-2) IgM antibodies and/or IgG antibodies" include anti-IgM antibodies and/or anti-IgG antibodies of an animal of the same species as the animal that produced the IgM antibodies and/or IgG antibodies.

The antibody may be of any isotype, for example, IgA, IgD, IgE, IgG (e.g., IgG1, IgG2, IgG3, or IgG4), IgM, or the like. The antibody may be a polyclonal antibody, a monoclonal antibody, a chimera antibody, or a single-chain antibody. It may also be a part of an antibody having antigen-binding properties (antibody fragment), such as a Fab fragment or a fragment produced by a Fab expression library. Examples of the antibody fragment include, but are not limited to, (i) Fab fragment, which is a monovalent fragment consisting of VL, VH, CL, and CH1 domains, (ii) an F(ab')₂ fragment which is a divalent fragment containing two Fab fragments linked by a disulfide crosslinking at the hinge region; (iii) an Fv fragment consisting of the single-armed VL and VH domains of an antibody; (iv) a Fab' fragment obtained by breaking the disulfide crosslinking of the F(ab')₂ fragment by using mild reducing conditions; (v) an Fv fragment (dsFv) stabilized by disulfide, and (vi) a domain antibody (dAb), which is a single variable region domain (VH or VL) polypeptide of an antibody that specifically binds to an antigen.

The anti-novel coronavirus (SARS-CoV-2) IgM antibodies and/or IgG antibodies to be used in the method of the present invention may be produced by mouse, guinea pig, hamster, goat, or rabbit. As the anti-novel coronavirus (SARS-CoV-2) IgM antibodies and/or IgG antibodies, commercially available ones can be used.

The test kit of the present invention contains the above-mentioned sensitizer for immunochromatographic assay of the present invention and a substance that specifically binds to a novel coronavirus (SARS-CoV-2) IgM antibody and/or IgG antibody in separate containers.

The test kit of the present invention may further contain a manual stating that the sensitizer for immunochromatographic assay of the present invention and a substance that specifically binds to a novel coronavirus (SARS-CoV-2) IgM antibody and/or IgG antibody can or should be used for testing for novel coronavirus infection (COVID-19).

The above-mentioned novel coronavirus infection (COVID-19) test kit can contain any carrier, for example, pharmaceutically acceptable carrier, stabilizer, buffer component, other therapeutic drug, supplement, and the like. The pharmaceutically acceptable carrier includes, but is not limited to, a diluent such as water and physiological saline. The novel coronavirus infection (COVID-19) test kit of the present invention may contain a sample diluent, a test strip for immunochromatographic assay, and an instrument for immunochromatographic assay.

The present invention provides a method for testing a test subject for the possibility of being infected with novel coronavirus (COVID-19), including measuring novel coronavirus (SARS-CoV-2) IgM antibodies and/or IgG antibodies in a biological sample derived from the test subject by performing an antigen-antibody reaction in the presence of the sensitizer for immunochromatographic assay of the present invention.

A biological sample that can be used in the method is, for example, blood but is not particularly limited as long as the novel coronavirus (SARS-CoV-2) IgM antibodies and/or IgG antibodies can be detected. As the "blood", blood derived from any tissue can be assumed, but peripheral blood is generally used because of the easiness of collection. As a method for collecting blood, a method known per se can be applied. The collected blood may be used as it is in the present method, but may be used in the present method as a liquid component (plasma) obtained by separating cell components (erythrocyte, leukocyte, platelet, and the like) by a method known per se, such as centrifugation, filtration, and the like. It is also possible to use the blood in this method as a liquid component (serum) obtained by coagulating the blood and separating platelets and coagulation factors.

In conducting the test method of the present invention, for example, a biological sample such as blood from a test subject is suspended in an extraction liquid, and an antigen is extracted from the biological sample liquid. Then, for example, when the above-mentioned instrument for immunochromatographic assay of (4) is used, a biological sample liquid treated with an extraction liquid is added dropwise onto the sample-dropping part. By doing so, the biological sample liquid is developed from the sample-dropping part to the sample-labeling part, and then onto the membrane, due to capillarity, in the presence of the sample diluent for immunochromatographic assay containing the sensitizer for immunochromatographic assay of the present invention.

The target antigen (e.g., novel coronavirus (SARS-CoV-2) IgM antibodies and/or IgG antibodies) in the biological sample liquid forms an immunocomplex with a labeling substance (e.g., anti-novel coronavirus (SARS-CoV-2) IgM antibodies and/or IgG antibodies) carried by the sample-labeling part when passing through the sample-labeling part, and is developed as it is to one end of the development membrane. Then, the complex and the substance (e.g., antigen for novel coronavirus (SARS-CoV-2) IgM antibodies and/or IgG antibodies) present on the test line of the development membrane (coated on the test line) cause an immunochemical reaction and bind to each other, and develop color, whereby a visible line appears on the development membrane. When the color develops in this way, the sample is positive, and when the target antigen in the biological sample liquid is a novel coronavirus (SARS-CoV-2) IgM antibody, the test subject can be determined to be infected with the novel coronavirus infection (COVID-19). When the target antigen in the biological sample liquid is a novel coronavirus (SARS-CoV-2) IgG antibody, the subject can be determined to be infected with or have been infected with a novel coronavirus infection (COVID-19) in the past. In addition, the intensity of the positivity can be determined by visually observing or measuring the degree of coloration with an appropriate analytical instrument (e.g., immunochromatographic reader, spectrophotometer). For example, when a fluorescent dye is used as the detection means of the labeled antibody, UV or the like is irradiated on the test line, and the presence or absence of coloration and whether the test line is positive or not can be determined based on the degree of coloration.

On the other hand, a control line is provided after the test line, and the antibody present (coated there) and the labeling substance (e.g., anti-novel coronavirus (SARS-CoV-2) IgM antibodies and/or IgG antibodies), that has not formed an immunocomplex, react immunochemically, bind, and develop color, from which it can be visually confirmed that the immunochromatography has been performed without any problems.

After the sample liquid has been developed and passed through the development membrane, the absorbent pad absorbs unreacted labeled antibody and the like. When the sample does not contain the target antigen (e.g., novel coronavirus (SARS-CoV-2) IgM antibodies and/or IgG antibodies), the test line does not develop color and only the control line develops color. In this case, the sample is negative and, when the target antigen in the biological sample liquid is a novel coronavirus (SARS-CoV-2) IgM antibody, the test subject is not determined to be infected with the novel coronavirus (COVID-19). When the target antigen in the biological sample liquid is a novel coronavirus (SARS-CoV-2) IgG antibody, the subject is not determined to be infected or have been infected in the past with novel coronavirus (COVID-19).

In one embodiment, a test subject is not determined to be infected or have been infected in the past with novel coronavirus (COVID-19) when he/she tests negative to a novel coronavirus (SARS-CoV-2) IgM antibody as the target antigen in a biological sample liquid, and negative to a novel coronavirus (SARS-CoV-2) IgG antibody as the target antigen in a biological sample liquid. A test subject can be determined to be infected with novel coronavirus (COVID-19) and in the initial stage of infection when he/she tests positive to a novel coronavirus (SARS-CoV-2) IgM antibody as the target antigen in a biological sample liquid, and negative to a novel coronavirus (SARS-CoV-2) IgG antibody as the target antigen in a biological sample liquid. A test subject can be determined to be infected with novel coronavirus (COVID-19) and to have been infected for a certain period of time when he/she tests positive to a novel coronavirus (SARS-CoV-2) IgM antibody as the target antigen in a biological sample liquid, and positive to a novel coronavirus (SARS-CoV-2) IgG antibody as the target antigen in a biological sample liquid. A test subject can be determined to have suffered from novel coronavirus infection (COVID-19) when he/she tests negative to a novel coronavirus (SARS-CoV-2) IgM antibody as the target antigen in a biological sample liquid, and positive to a novel coronavirus (SARS-CoV-2) IgG antibody as the target antigen in a biological sample liquid.

A biological sample to be subjected to the method for testing of the present invention is a sample derived from a mammalian test subject. The mammal is not particularly limited as long as it is a mammal that may be infected with the novel coronavirus (COVID-19). Among them, rodents such as mouse, rat, hamster, guinea pig, and the like, experimental animals such as rabbit and the like, domestic animals such as swine, bovine, goat, horse, sheep, mink, and the like, pets such as dog, cat, and the like, primates such as human, monkey, Macaca fascicularis, Macaca mulatta, marmoset, orangutan, chimpanzee, and the like are preferred, and human is particularly preferred.

The method for treating a novel coronavirus infection (COVID-19) of the present invention includes at least the following steps (1) and (2):
(1) a step of measuring a novel coronavirus (SARS-CoV-2) by conducting an antigen-antibody reaction in the presence of a sensitizer for immunochromatographic assay, containing the aforementioned polymer or copolymer, and
(2) a step of treating a patient with a novel coronavirus infection (COVID-19) based on the assay results of the above-mentioned (1).

Patients include those who may be infected with the novel coronavirus (SARS-CoV-2). Note that assay means detecting whether or not novel coronavirus (SARS-CoV-2) IgM antibodies and/or IgG antibodies are/is present in samples derived from patients.

The process of treating patients for novel coronavirus infections includes not only administering known therapeutic drugs, therapeutic drugs to be marketed hereafter, and therapeutic drug candidates in clinical stages, but also symptomatic treatment (administration of aspirin, administration of fluid replacement·infusion, and the like).

The method for treating a novel coronavirus infection (COVID-19) of the present invention can detect novel coronavirus (SARS-CoV-2) IgM antibodies and/or IgG antibodies with high sensitivity, and thus can rapidly treat novel coronavirus infection (COVID-19) of patients with novel coronavirus infection (COVID-19).

Symptoms of novel coronavirus infection (COVID-19) range from asymptomatic infection to severe pneumonia and death. Specifically, fever, dry cough, malaise·lassitude, sputum, olfactory disorder·taste disorder, short breath, muscular pain·arthralgia, sore throat, headache, chills, nausea·vomiting, nasal congestion, diarrhea, hemoptysis, conjunctival hyperemia, and the like can be mentioned.

In another embodiment, the method for treating a novel coronavirus infection (COVID-19) of the present invention includes at least the following steps (1) and (2):
(1) a step of measuring a novel coronavirus (SARS-CoV-2) by conducting an antigen-antibody reaction in the presence of a sensitizer for immunochromatographic assay, containing the aforementioned polymer or copolymer, and
(2) a step of treating a test subject other than human with a novel coronavirus infection (COVID-19) based on the assay results of the above-mentioned (1).

Test subjects other than human that are subjected to the treatment method of the present invention are not limited but preferably mean mammals. Further preferably, they are mammals that may be infected with the novel coronavirus (COVID-19). Among them, rodents such as mouse, rat, hamster, guinea pig, and the like, experimental animals such as rabbit and the like, domestic animals such as swine, bovine, goat, horse, sheep, mink, and the like, pets such as dog, cat, and the like, primates such as monkey, Macaca fascicularis, Macaca mulatta, marmoset, orangutan, chimpanzee, and the like are preferred.

The present invention provides a kit for the treatment of a novel coronavirus infection (COVID-19), containing a sensitizer for immunochromatographic assay containing the polymer or copolymer represented by the above-mentioned formula [1], [3], [4], or [5].

Examples of the therapeutic agent include those containing a therapeutic drug for the novel coronavirus infection (COVID-19). While the therapeutic drug is not limited, remdesivir, dexamethasone, favipiravir (Avigan), ciclesonide, nafamostat, camostat, ivermectin, nelfinavir, and the like can be mentioned. The therapeutic drug may be tocilizumab, baricitinib, acalabrutinib, ravulizumab, eritoran, ibudilast, LY3127804, otilimab, HLCM051, ADR-001, icatibant, apremilast, or cenicriviroc, which are therapeutic drugs for severe pneumonia and acute respiratory distress syndrome. The therapeutic drug may be cacilibimab, imdevimab, and/or a mixture thereof, which are virus-neutralizing antibodies that exhibit neutralizing activity against SARS-CoV-2 by noncompetitively binding to the receptor binding site of the spike protein of the virus. It may be a therapeutic drug to be marketed hereafter or a therapeutic drug candidate in clinical stages. The therapeutic agent may contain aspirin, fluid replacement·infusion, and the like that are used for symptomatic therapy.

The treatment kit of the present invention contains the above-mentioned sensitizer for immunochromatographic assay of the present invention and a therapeutic agent for novel coronavirus infection (COVID-19) in separate containers.

The treatment kit of the present invention may further contain a manual stating that the sensitizer for immunochromatographic assay of the present invention and a therapeutic agent for novel coronavirus infection (COVID-19) can or should be used for treating novel coronavirus infection (COVID-19).

The above-mentioned novel coronavirus infection (COVID-19) treatment kit can contain any carrier, for example, pharmaceutically acceptable carrier, stabilizer, buffer component, other therapeutic drug, supplement, and the like. The pharmaceutically acceptable carrier includes, but is not limited to, a diluent such as water and physiological saline. The novel coronavirus infection (COVID-19) treatment kit of the present invention may contain a sample diluent, a test strip for immunochromatographic assay, and an instrument for immunochromatographic assay.

The treatment kit of the present invention may further contain a substance that specifically binds to a novel coronavirus (SARS-CoV-2) IgM antibody and/or IgG antibody.

The kit for treating a novel coronavirus infection (COVID-19) of the present invention can detect novel coronavirus (SARS-CoV-2) IgM antibodies and/or IgG antibodies with high sensitivity, and thus can rapidly treat novel coronavirus infection (COVID-19) of test subjects (patients) with novel coronavirus infection (COVID-19).

The present invention is explained in further detail in the following by referring to Examples which are not to be construed as limitative.

### [Example]

### Production Example

### (Synthesis of copolymers of the present invention and Comparative Example)

A copolymer used as a sensitizer for the immunochromatographic assay of the present invention and a copolymer of Comparative Example were synthesized. The details are as follows.

The polymers used in the present invention and Comparative Example are 2-(methacryloyloxy)ethyl 2'-(trimethylammonio)ethyl phosphate (MPC) polymers and random copolymers synthesized from MPC and the following monomers.
(polymer 1)
   weight average molecular weight; 1030×10³, MPC polymer
(polymer 2)
   weight average molecular weight; 600×10³, ratio of number of constitutional units based on MPC monomer and number of constitutional units based on butyl methacrylate; 80:20
(polymer 3)
   weight average molecular weight; 1210×10³, ratio of number of constitutional units based on MPC monomer and number of constitutional units based on butyl methacrylate; 80:20
(polymer 4)
   weight average molecular weight; 22×10³, ratio of number of constitutional units based on MPC monomer and number of constitutional units based on butyl methacrylate and number of constitutional units based on glycerol methacrylate based on constitutional unit number butyl methacrylate; 40:40:20
(polymer 5)
   weight average molecular weight; 26×10³, ratio of number of constitutional units based on MPC monomer and number of constitutional units based on butyl methacrylate and number of constitutional units based on glycerol methacrylate; 40:20:40
(polymer 6)
   weight average molecular weight; 680×10³, ratio of number of constitutional units based on MPC monomer and number of constitutional units based on methacrylic acid; 30:70

### Example

### (Confirmation of detection sensitivity using known instrument for immunochromatographic assay)

In this Example, whether the above-mentioned polymers can improve the detection sensitivity of known detection kits for novel coronavirus (SARS-CoV-2) IgM antibodies and/or IgG antibodies (presence of sensitizing effect) was confirmed. The details are as follows.

### (Comparative Example)

Evaluation was made using commercially available novel coronavirus (SARS-CoV-2) IgM antibody detection kit Novel Coronavirus (SARS-CoV-2) IgM Test Kit (Colloidal Gold) (manufactured by Ray Biotec.) and SARS-CoV-2 IgG antibody detection kit Novel Coronavirus (SARS-CoV-2) IgG Test Kit (Colloidal Gold) (manufactured by Ray Biotec.). More specifically, novel coronavirus-positive patients' serum (CoV-PosM-S-100, manufactured by RayBiotec.) (25 µL) was added to the diluent (245 µL) in the SARS-CoV-2 IgM antibody detection kit or SARS-CoV-2 IgG antibody detection kit, and the diluent was developed on an immunochromatographic kit.

### (Example 1)

Polymer 1, 2, 3, 4, or 5 was added to the diluent of the kit to a final concentration of 0.5 wt%. Furthermore, a solution prepared by diluting the novel coronavirus-positive patient serum 100-fold that of Comparative Example was developed, and the detection sensitivity was compared.

### (Example 2)

Polymer 1, 2, or 4 was added to the diluent of the kit to a final concentration of 0.5 wt%, and then polymer 6 was further added to a final concentration of 0.5 wt%. Other than that, the detection sensitivity was compared in the same manner as in Example 1.

### (Example 3)

Polymer 1, 2, 3, 4, or 5 was carried on the sample pad of the kit at a carrying amount of 5 mg, and a solution prepared by diluting the novel coronavirus-positive patient serum 100-fold that of Comparative Example was developed, and the detection sensitivity was compared.

### (Example 4)

Polymer 1, 2, or 4 was carried on the sample pad of the kit at a carrying amount of 5 mg, and polymer 6 was further carried at a carrying amount of 5 mg. Other than that, the detection sensitivity was compared in the same manner as in Example 3.

### (Example 5)

Polymer 1, 2, 3, 4, or 5 was carried on the conjugate pad of the kit at a carrying amount of 500 µg, and a solution prepared by diluting the novel coronavirus-positive patient serum 100-fold that of Comparative Example was developed, and the detection sensitivity was compared.

### (Example 6)

Polymer 1, 2, or 4 was carried on the conjugate pad of the kit at a carrying amount of 500 µg, and polymer 6 was further carried at a carrying amount of 500 µg. Other than that, the detection sensitivity was compared in the same manner as in Example 5.

### (Confirmation results of IgM antibody detection sensitivity using instrument for immunochromatographic assay)

According to Examples 1 and 2, the improvement in detection sensitivity by the addition of polymer to diluent was measured using a known novel coronavirus (SARS-CoV-2) IgM antibody detection kit Novel Coronavirus (SARS-CoV-2) IgM Test Kit (Colloidal Gold) (manufactured by Ray Biotec.), and the results are shown in Table 1.

**[Table 1]**

| name in Description | image | presence or absence of sensitizer | dilution ratio | detection line |
|---|---|---|---|---|
| Comparative Example | 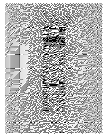 | not contained | 1 fold | standard |
| (Comparative Example 100-fold dilution) | 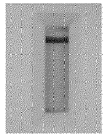 | not contained | 100 folds | almost invisible (not distinguished) |
| polymer 1 | 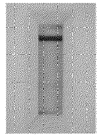 | contained | 100 folds | rather thin (compared with standard) |
| polymer 2 | 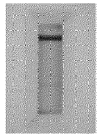 | contained | 100 folds | rather thin (compared with standard) |
| polymer 3 | 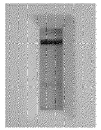 | contained | 100 folds | rather thin (compared with standard) |
| polymer 4 | 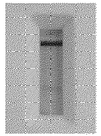 | contained | 100 folds | rather thin (compared with standard) |
| polymer 5 | 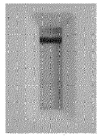 | contained | 100 folds | rather thin (compared with standard) |
| polymer 1 | 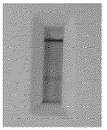 | contained | 100 folds | equal to or greater than (compared with standard) |
| polymer 6 | | | | |
| polymer 2 | 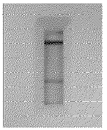 | contained | 100 folds | equal to or greater than (compared with standard) |
| polymer 6 | | | | |
| polymer 4 | 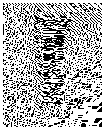 | contained | 100 folds | equal to or greater than (compared with standard) |
| polymer 6 | | | | |

When 0.5 wt% of polymer 1, 2, 3, 4, or 5 was added, a test line with a density equal to or higher, rather thinner than that of the control as a comparative example could be detected for IgM. Thus, it was confirmed that the addition of polymer 1, 2, 3, 4, or 5 improved the sensitivity. It was preferably confirmed that the combined use of polymer 6 further improved the sensitivity.

### (Confirmation results of IgG antibody detection sensitivity using instrument for immunochromatographic assay)

According to Examples 1 and 2, the improvement in detection sensitivity by the addition of polymer to diluent was measured using a known novel coronavirus (SARS-CoV-2) IgG antibody detection kit Novel Coronavirus (SARS-CoV-2) IgG Test Kit (Colloidal Gold) (manufactured by Ray Biotec.), and the results are shown in Table 2.

**[Table 2]**

| name in Description | image | presence or absence of sensitizer | dilution ratio | detection line |
|---|---|---|---|---|
| Comparative Example | 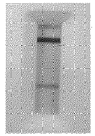 | not contained | 1 fold | standard |
| (Comparative Example 100-fold dilution) | 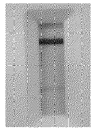 | not contained | 100 folds | thin (difficult to distinguish) |
| polymer 1 | 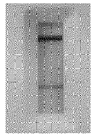 | contained | 100 folds | equal to or greater than (compared with standard) |
| polymer 2 | 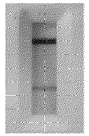 | contained | 100 folds | equal to or greater than (compared with standard) |
| polymer 3 | 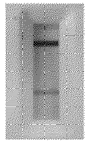 | contained | 100 folds | equal to or greater than (compared with standard) |
| polymer 4 | 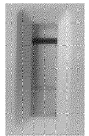 | contained | 100 folds | rather thin (compared with standard) |
| polymer 5 | 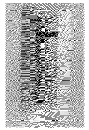 | contained | 100 folds | rather thin (compared with standard) |
| polymer 1 | 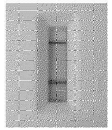 | contained | 100 folds | equal to or greater than (compared with standard) |
| polymer 6 | | | | |
| polymer 2 | 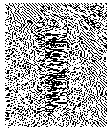 | contained | 100 folds | equal to or greater than (compared with standard) |
| polymer 6 | | | | |
| polymer 4 | 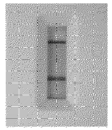 | contained | 100 folds | equal to or greater than (compared with standard) |
| polymer 6 | | | | |

When 0.5 wt% of polymer 1, 2, 3, 4, or 5 was added, a test line with a density equal to or higher, or rather thinner than that of the control as a comparative example could be detected for IgG. Thus, it was confirmed that the addition of polymer 1, 2, 3, 4, or 5 improved the sensitivity. It was preferably confirmed that the combined use of polymer 6 further improved the sensitivity.

According to Examples 3 and 4, the improvement in detection sensitivity by carrying polymer on a sample pad was measured using a known novel coronavirus (SARS-CoV-2) IgM antibody detection kit Novel Coronavirus (SARS-CoV-2) IgM Test Kit (Colloidal Gold) (manufactured by Ray Biotec.), and the results are shown in Table 3.

**[Table 3]**

| name in Description | image | presence or absence of sensitizer | dilution ratio | detection line |
|---|---|---|---|---|
| Comparative Example | 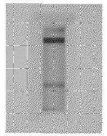 | not contained | 1 fold | standard |
| (Comparative Example 100-fold dilution) | 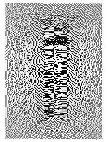 | not contained | 100 folds | almost invisible (not distinguished) |
| polymer 1 | 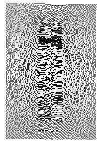 | contained | 100 folds | rather thin (compared with standard) |
| polymer 2 | 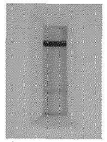 | contained | 100 folds | rather thin (compared with standard) |
| polymer 3 | 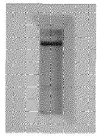 | contained | 100 folds | rather thin (compared with standard) |
| polymer 4 | 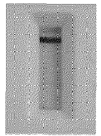 | contained | 100 folds | rather thin (compared with standard) |
| polymer 5 | 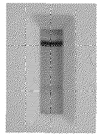 | contained | 100 folds | rather thin (compared with standard) |
| polymer 1 | 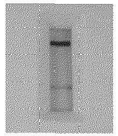 | contained | 100 folds | equal to or greater than (compared with standard) |
| polymer 6 | | | | |
| polymer 2 | 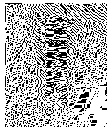 | contained | 100 folds | equal to or greater than (compared with standard) |
| polymer 6 | | | | |
| polymer 4 | 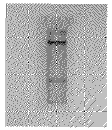 | contained | 100 folds | equal to or greater than (compared with standard) |
| polymer 6 | | | | |

When polymer 1, 2, 3, 4, or 5 was carried on a sample pad, a test line with a density equal to or higher or rather thinner than that of the control as a comparative example could be detected for IgM. Thus, it was confirmed that carrying of polymer 1, 2, 3, 4, or 5 on a sample pad improved the sensitivity. It was preferably confirmed that the combined use of polymer 6 further improved the sensitivity.

According to Examples 3 and 4, the improvement in detection sensitivity by carrying polymer on a sample pad was measured using a known novel coronavirus (SARS-CoV-2) IgG antibody detection kit Novel Coronavirus (SARS-CoV-2) IgG Test Kit (Colloidal Gold) (manufactured by Ray Biotec.), and the results are shown in Table 4.

**[Table 4]**

| name in Description | image | presence or absence of sensitizer | dilution ratio | detection line |
|---|---|---|---|---|
| Comparative Example | 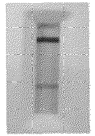 | not contained | 1 fold | standard |
| (Comparative Example 100-fold dilution) | 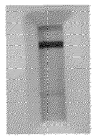 | not contained | 100 folds | thin (difficult to distinguish) |
| polymer 1 | 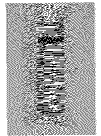 | contained | 100 folds | equal to or greater than (compared with standard) |
| polymer 2 | 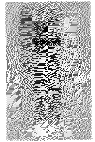 | contained | 100 folds | equal to or greater than (compared with standard) |
| polymer 3 | 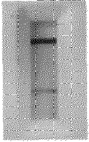 | contained | 100 folds | equal to or greater than (compared with standard) |
| polymer 4 | 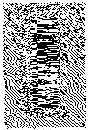 | contained | 100 folds | equal to or greater than (compared with standard) |
| polymer 5 | 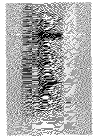 | contained | 100 folds | rather thin (compared with standard) |
| polymer 1 | 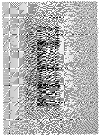 | contained | 100 folds | equal to or greater than (compared with standard) |
| polymer 6 | | | | |
| polymer 2 | 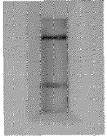 | contained | 100 folds | equal to or greater than (compared with standard) |
| polymer 6 | | | | |
| polymer 4 | 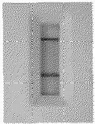 | contained | 100 folds | equal to or greater than (compared with standard) |
| polymer 6 | | | | |

When polymer 1, 2, 3, 4, or 5 was carried on a sample pad, a test line with a density equal to or higher or rather thinner than that of the control as a comparative example could be detected for IgG. Thus, it was confirmed that carrying of polymer 1, 2, 3, 4, or 5 on a sample pad improved the sensitivity. It was preferably confirmed that the combined use of polymer 6 further improved the sensitivity.

According to Examples 5 and 6, the improvement in detection sensitivity by carrying polymer on a conjugate pad was measured using a known novel coronavirus (SARS-CoV-2) IgM antibody detection kit Novel Coronavirus (SARS-CoV-2) IgM Test Kit (Colloidal Gold) (manufactured by Ray Biotec.), and the results are shown in Table 5.

**[Table 5]**

| name in Description | image | presence or absence of sensitizer | dilution ratio | detection line |
|---|---|---|---|---|
| Comparative Example | 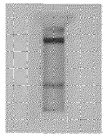 | not contained | 1 fold | standard |
| (Comparative Example 100-fold dilution) | 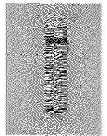 | not contained | 100 folds | almost invisible (not distinguished) |
| polymer 1 | 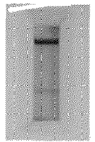 | contained | 100 folds | rather thin (compared with standard) |
| polymer 2 | 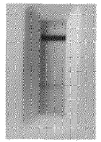 | contained | 100 folds | rather thin (compared with standard) |
| polymer 3 | 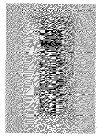 | contained | 100 folds | rather thin (compared with standard) |
| polymer 4 | 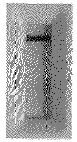 | contained | 100 folds | rather thin (compared with standard) |
| polymer 5 | 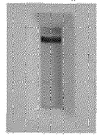 | contained | 100 folds | rather thin (compared with standard) |
| polymer 1 | 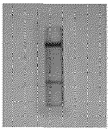 | contained | 100 folds | equal to or greater than (compared with standard) |
| polymer 6 | | | | |
| polymer 2 | 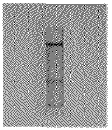 | contained | 100 folds | equal to or greater than (compared with standard) |
| polymer 6 | | | | |
| polymer 4 | 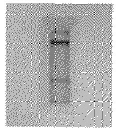 | contained | 100 folds | equal to or greater than (compared with standard) |
| polymer 6 | | | | |

When polymer 1, 2, 3, 4, or 5 was carried on a conjugate pad, a test line with a density equal to or higher or rather thinner than that of the control as a comparative example could be detected for IgM. Thus, it was confirmed that carrying of polymer 1, 2, 3, 4, or 5 on a conjugate pad improved the sensitivity. It was preferably confirmed that the combined use of polymer 6 further improved the sensitivity.

According to Examples 5 and 6, the improvement in detection sensitivity by carrying polymer on a conjugate pad was measured using a known novel coronavirus (SARS-CoV-2) IgG antibody detection kit Novel Coronavirus (SARS-CoV-2) IgG Test Kit (Colloidal Gold) (manufactured by Ray Biotec.), and the results are shown in Table 6.

**[Table 6]**

| name in Description | image | presence or absence of sensitizer | dilution ratio | detection line |
|---|---|---|---|---|
| Comparative Example | 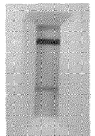 | not contained | 1 fold | standard |
| (Comparative Example 100-fold dilution) | 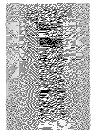 | not contained | 100 folds | thin (difficult to distinguish) |
| polymer 1 | 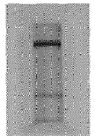 | contained | 100 folds | equal to or greater than (compared with standard) |
| polymer 2 | 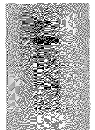 | contained | 100 folds | equal to or greater than (compared with standard) |
| polymer 3 | 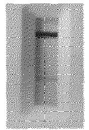 | contained | 100 folds | rather thin (compared with standard) |
| polymer 4 | 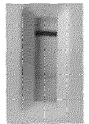 | contained | 100 folds | rather thin (compared with standard) |
| polymer 5 | 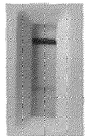 | contained | 100 folds | rather thin (compared with standard) |
| polymer 1 | 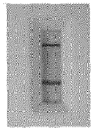 | contained | 100 folds | equal to or greater than (compared with standard) |
| polymer 6 | | | | |
| polymer 2 | 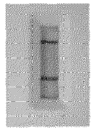 | contained | 100 folds | equal to or greater than (compared with standard) |
| polymer 6 | | | | |
| polymer 4 | | contained | 100 folds | equal to or greater than (compared with standard) |
| polymer 6 | 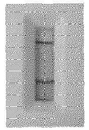 | | | |

When polymer 1, 2, 3, 4, or 5 was carried on a conjugate pad, a test line with a density equal to or higher or rather thinner than that of the control as a comparative example could be detected for IgG. Thus, it was confirmed that carrying of polymer 1, 2, 3, 4, or 5 on a conjugate pad improved the sensitivity. It was preferably confirmed that the combined use of polymer 6 further improved the sensitivity.

From the above Examples, the present invention was able to provide an immunochromatographic assay that has a markedly superior sensitizing effect as compared with known immunochromatographic assays for novel coronavirus (SARS-CoV-2) IgM antibodies and/or IgG antibodies as assay target substances.

As is clear from those described above, the present invention was able to provide an immunochromatographic assay that has a superior sensitizing effect as compared with known immunochromatographic assays for novel coronavirus (SARS-CoV-2) IgM antibodies and/or IgG antibodies as assay target substances.

Furthermore, the method for treating novel coronavirus infection (COVID-19) of the present invention can detect the novel coronavirus (SARS-CoV-2) with high sensitivity. Thus, the therapeutic effect is high because patients with novel coronavirus infection or patients who may be infected with the novel coronavirus (SARS-CoV-2) can rapidly receive a treatment for the novel coronavirus infection.

### [Industrial Applicability]

The present invention can provide a sensitizer for immunochromatographic assay for novel coronavirus (SARS-CoV-2) IgM antibodies and/or IgG antibodies as assay target substances.

This application is based on a patent application No. 2020-206166 filed in Japan (filing date: December 11, 2020) and a patent application No. 2021-006659 filed in Japan (filing date: January 19, 2021), the contents of which are incorporated in full herein.

## Claims

1. A sensitizer for immunochromatographically assaying novel coronavirus (SARS-CoV-2) IgM antibodies and/or IgG antibodies as assay target substances, comprising a polymer represented by the following formula [4]: 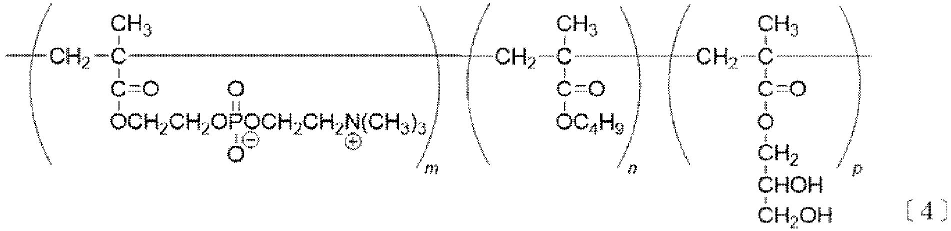 wherein m, n, and p are each a constitutional unit number, and m:n:p is 100 to 30:0 to 70:0 to 50.

2. A sensitizer for immunochromatographically assaying novel coronavirus (SARS-CoV-2) IgM antibodies and/or IgG antibodies as assay target substances, comprising a polymer represented by the following formula [3]: 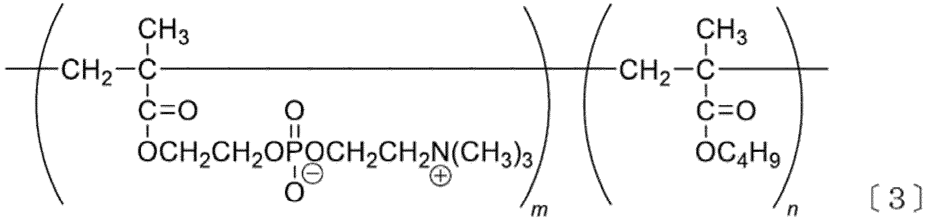 wherein m and n are each a constitutional unit number, and m:n is 100 to 30:0 to 70.

3. A sample diluent for immunochromatographically assaying novel coronavirus (SARS-CoV-2) IgM antibodies and/or IgG antibodies as assay target substances, comprising the sensitizer for immunochromatographic assay according to claim 1.

4. A method for immunochromatographically assaying novel coronavirus (SARS-CoV-2) IgM antibodies and/or IgG antibodies as assay target substances, comprising conducting an antigen-antibody reaction in the presence of the sensitizer for immunochromatographic assay according to claim 1.

5. An instrument for immunochromatographically assaying novel coronavirus (SARS-CoV-2) IgM antibodies and/or IgG antibodies as assay target substances, comprising the sensitizer for immunochromatographic assay according to claim 1.

6. An instrument for immunochromatographically assaying novel coronavirus (SARS-CoV-2) IgM antibodies and/or IgG antibodies as assay target substances, comprising the sensitizer for immunochromatographic assay according to claim 1, wherein the sensitizer is carried on a development membrane, a sample pad, or a conjugate pad.

7. A test kit for novel coronavirus infection (COVID-19), comprising the sensitizer for immunochromatographic assay according to claim 1 and a substance that specifically binds to a novel coronavirus (SARS-CoV-2) IgM antibody and/or IgG antibody.

8. A method for testing a test subject for the possibility of being infected with a novel coronavirus (COVID-19), comprising measuring novel coronavirus (SARS-CoV-2) IgM antibodies and/or IgG antibodies in a biological sample derived from the test subject by conducting an antigen-antibody reaction in the presence of the sensitizer for immunochromatographic assay according to claim 1.

9. A method for treating a novel coronavirus infection (COVID-19), comprising the following steps (1) and (2):
(1) a step of measuring a novel coronavirus (SARS-CoV-2) by conducting an antigen-antibody reaction in the presence of a sensitizer for immunochromatographic assay comprising a polymer represented by the following formula [4], and
(2) a step of treating a patient with a novel coronavirus infection (COVID-19) based on the assay results of the above-mentioned (1): 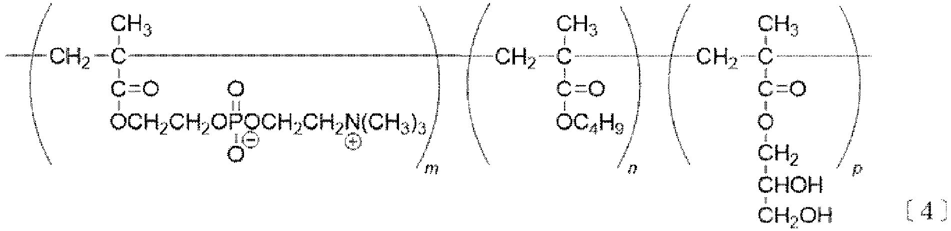 wherein m, n, and p are each a constitutional unit number, and m:n:p is 100 to 30:0 to 70:0 to 50.

10. A kit for the treatment of a novel coronavirus infection (COVID-19), comprising a sensitizer for immunochromatographic assay comprising a polymer represented by the following formula [4] and a therapeutic agent for the novel coronavirus infection (COVID-19): 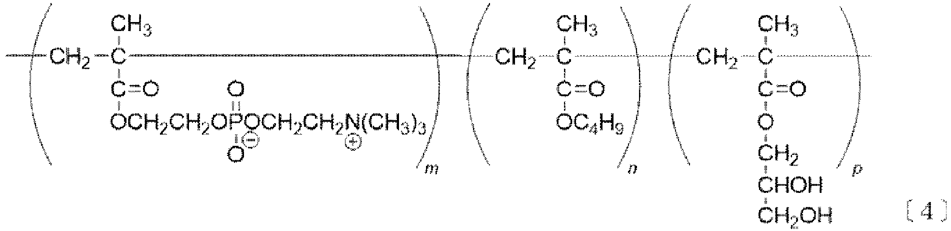 wherein m, n, and p are each a constitutional unit number, and m:n:p is 100 to 30:0 to 70:0 to 50.

11. A sensitizer for immunochromatographically assaying novel coronavirus (SARS-CoV-2) IgM antibodies and/or IgG antibodies as assay target substances, comprising a polymer represented by the following formula [4] and a copolymer represented by the following formula [5]: 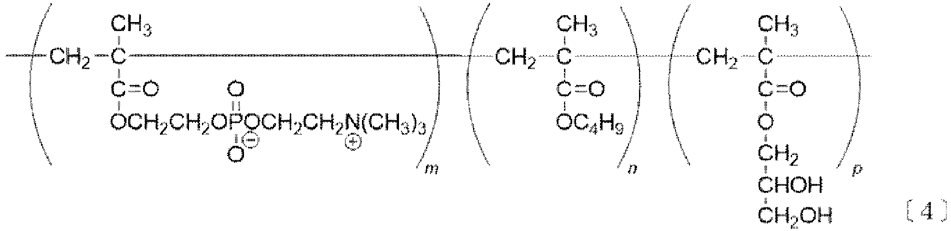 wherein m, n, and p are each a constitutional unit number, and m:n:p is 100 to 30:0 to 70:0 to 50, 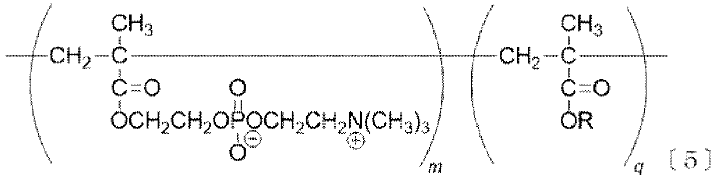 wherein R in the number of q are each independently a hydrogen atom or a cation, m and q are each a constitutional unit number, and m:q is 99 to 20:1 to 80.
